# EUROPÄISCHE PATENTSCHRIFT

(11) **EP 3 415 940 B1**
(45) Veröffentlichungstag und Bekanntmachung des Hinweises auf die Patenterteilung: **31.07.2019**
(21) Anmeldenummer: 17176031.7
(22) Anmeldetag: 14.06.2017
(51) Int. Cl.: G01R 33/422, G01R 33/34, H01Q 1/40, G01R 33/20, A61B 5/05

(54) **MR-HOCHFREQUENZSCHIRMEINHEIT.**
MR-HIGH FREQUENCY SHIELDING UNIT.
UNITÉ DE PROTECTION HAUTE FRÉQUENCE POUR RÉSONNACE MAGNÉTIQUE.

(43) Veröffentlichungstag der Anmeldung: 19.12.2018
(73) Patentinhaber: Siemens Healthcare GmbH, 91052 Erlangen (DE)
(72) Erfinder: Eberler, Ludwig, 92318 Neumarkt i.d.OPf. (DE); Nistler, Jürgen, 91056 Erlangen (DE); Vester, Markus, 90471 Nürnberg (DE)

(56) Entgegenhaltungen:
- EP-A1- 2 175 289
- WO-A1-91/19994
- "CARBON FIBER RADIOFREQUENCY SHIELD FOR MAGNETIC RESONANCE IMAGING", IP.COM JOURNAL, IP.COM INC., WEST HENRIETTA, NY, US, 20. Juni 2014 (2014-06-20), XP013163148, ISSN: 1533-0001

## Beschreibung

Die Erfindung betrifft eine Hochfrequenzschirmeinheit zur Schirmung einer Hochfrequenzantenneneinheit einer Magnetresonanzvorrichtung sowie eine Magnetresonanzvorrichtung.

Die Magnetresonanztomographie (MRT; engl. Magnetic Resonance Imaging, MRI) ist eine bekannte Technik zum Erzeugen von Magnetresonanzabbildungen eines Patienten, die auf dem physikalischen Phänomen der Magnetresonanz (MR) beruht. Dazu werden während einer Magnetresonanzmessung mit einer Hochfrequenzantenneneinheit um Auslösen von Magnetresonanzsignalen Anregungspulse in den Patienten eingestrahlt. Zur Ortskodierung der Magnetresonanzsignale umfasst die Magnetresonanzvorrichtung ferner eine Gradientenspuleneinheit, mit der Magnetfeldgradienten erzeugt werden. Die Magnetfeldgradienten werden einem statischen Hauptmagnetfeld B₀ überlagert, welches durch einen Hauptmagneten erzeugt wird.

Als Hochfrequenzantenneneinheit wird in der Regel eine fest in der Magnetresonanzvorrichtung eingebaute Sendeantenne verwendet, die oftmals Ganzkörper-Hochfrequenzspule oder kurz Köperspule (engl. body coil) bezeichnet wird. Dabei ist es üblich, die eigentliche Hochfrequenzantenneneinheit durch eine Hochfrequenzschirmeinheit, oftmals auch als Hochfrequenzschirm, HF-Schirm oder Schirm bezeichnet, zu beranden. Verschiedene Ausführungsbeispiele von Hochfrequenzschirmeinheiten sind in den Druckschriften US 7501823 B2, US 8766632 B2 und US 20130300418 A1 beschrieben.

Eine Hochfrequenzschirmeinheit umfasst üblicherweise eine Massefläche und damit auch eine Fläche für Spiegelströme der Hochfrequenzantenneneinheit. Üblicherweise ist die Hochfrequenzschirmeinheit zwischen der Hochfrequenzantenneneinheit und der Gradientenspuleneinheit angeordnet, die gegenüber einem Patientenaufnahmebereich in der Regel weiter außen angeordnet ist. Oftmals wird die Hochfrequenzschirmeinheit unmittelbar auf einer Innenseite der Gradientenspuleneinheit aufgebracht.

Einerseits sollte die Hochfrequenzschirmeinheit hochfrequente Ströme möglichst gut leiten, um einen optimalen Wirkungsgrad der Hochfrequenzantenneneinheit zu gewährleisten. Andererseits sollen aber niederfrequente Wirbelströme, die üblicherweise durch Felder der Gradientenspuleneinheit induziert werden, möglichst minimiert werden, um eine Erwärmung der Hochfrequenzschirmeinheit zu unterbinden. Ferner könnten die Felder der Wirbelströme auch bis in den Patienten reichen und dort bei einer Magnetresonanzmessung zu Bildstörungen und/oder Bildartefakten führen.

Ein weiteres Problem können störende Einkopplungen, z.B. in Form von Feldspitzen (engl. spikes), darstellen, die beispielsweise durch Schalttakte von Gradientenverstärkern hervorgerufen werden können. Damit verbundene Störungen sollten ebenso durch die Hochfrequenzschirmeinheit gedämpft werden, um die Bildgebung nicht zu beeinträchtigen.

Zur gleichzeitigen Erfüllung der Anforderungen (hohe Effizienz der Sendeantenne, ausreichende Unterdrückung von Wirbelströmen und ausreichende Dämpfung von Störungen) setzt man seit langem Hochfrequenzschirmeinheiten ein, die aus drei Schichten aufgebaut sind, nämlich aus zwei Leitschichten und einer dazwischen liegenden Zwischenschicht, wobei die Zwischenschicht gleichzeitig als Isolationsschicht und als Trägerschicht dient. Beispielhaft ist eine solche Anordnung in der Druckschrift WO 91/19994 A1 offenbart.

EP2175289 offenbart eine Elektromagnetische Abschirmung der Kernresonanz und eine Abschirmung der elektrischen Schaltung . Das Abschirmmaterial umfasst ein leitfähiges Material (Zinnverbindung). Eine Leiterplatte liegt zwischen zwei Abschirmschichten. Die Abschirmschichtdicke liegt zwischen 5 und 50 um.

Hochfrequenzschirmeinheiten können insbesondere aus Leiterplattenmaterial mit beidseitig aufgebrachten Kupferlagen bestehen, d.h. die Vorder- und die Rückseite des Leiterplattenmaterials ist mit Kupfer beschichtet. Beide Kupferlagen tragen zur Schirmwirkung der Hochfrequenzschirmeinheit bei.

Derart ausgebildete Hochfrequenzschirmeinheiten haben sich in der Vergangenheit gut geeignet für Magnetresonanzvorrichtungen mit Stärken des Hauptmagnetfelds B₀ von typischerweise 1,5 oder 3 T. Jedoch zeigen derartige Hochfrequenzschirmeinheiten für Magnetresonanzvorrichtungen mit niedrigen Stärken des Hauptmagnetfelds B₀, insbesondere bei weniger als 1 T, deutlich höhere Verluste und deutlich geringere Dämpfwirkungen auf Störungen von außen.

Als Aufgabe der vorliegenden Erfindung kann die Bereitstellung einer Hochfrequenzschirmeinheit angesehen werden, die sich besonders für Magnetresonanzvorrichtungen mit niedrigem Hauptmagnetfeld B₀ eignet.

### Zusammenfassung

Die Aufgabe wird durch die Merkmale der unabhängigen Ansprüche gelöst. Vorteilhafte Ausgestaltungen sind in den Unteransprüchen beschrieben.

Demnach wird eine Hochfrequenzschirmeinheit zur Schirmung einer Hochfrequenzantenneneinheit einer Magnetresonanzvorrichtung vorgeschlagen. Die Hochfrequenzschirmeinheit umfasst zumindest folgende vier Schichten (engl. layer): eine Trägerschicht, eine erste Leitschicht, eine Isolationsschicht und eine zweite Leitschicht. Dabei sind die erste Leitschicht zwischen der Trägerschicht und der Isolationsschicht sowie die Isolationsschicht zwischen der ersten Leitschicht und der zweiten Leitschicht angeordnet.

Die Isolationsschicht weist eine Schichtdicke von höchstens 50 µm, insbesondere höchstens 20 µm, insbesondere höchstens 10 µm auf.

Die Isolationsschicht ist zumindest teilweise durch ein Sprühverfahren und/oder ein Druckverfahren, insbesondere ein Siebdruckverfahren, auf der ersten Leitschicht aufgebracht. Mittels Sprühverfahren kann die Isolationsschicht besonders einfach aufbracht werden. Mittels Druckverfahren kann die Isolationsschicht besonders einfach aufbracht werden.

Unter eine Schicht wird hier vorzugsweise ein flächig ausgebildeter Bestandteil der Hochfrequenzschirmeinheit verstanden. Eine Schicht weist üblicherweise eine, insbesondere flächige, Schichtebene auf, wobei hier die Schichtebene durchaus auch gekrümmt ausgebildet sein kann. Ferner weist eine Schicht parallel zur Schichtebene eine laterale Ausdehnung und senkrecht zur Schichtebene eine senkrechte Ausdehnung auf, die auch als Schichtdicke bezeichnet werden kann. Dabei ist die laterale Ausdehnung einer Schicht üblicherweise wesentlich größer, insbesondere mehr als 1000 Mal größer, als ihre senkrechte Ausdehnung.

Vorzugsweise sind die Trägerschicht, die erste Leitschicht, die Isolationsschicht und die Leitschicht fest und/oder direkt, insbesondere auf atomarer und/oder molekularer Ebene, miteinander verbunden, d.h. sie bilden einen festen und/oder direkten Schichtverbund. Beispielsweise werden die Schichten nacheinander mittels Beschichtungsverfahren nacheinander ausgehend von der Trägerschicht aufeinander aufgebracht, so dass schließlich der vorgeschlagene Schichtaufbau entsteht, nämlich Trägerschicht/erste Leitschicht/Isolationsschicht/zweite Leitschicht.

Die Trägerschicht verleiht der Hochfrequenzschirmeinheit vorteilhafterweise eine ausreichende Stabilität und/oder Robustheit, z.B. gegenüber etwaigen mechanischen Belastungen, so dass Beschädigungen vermieden werden können. Außerdem erlaubt die Trägerschicht vorteilhafterweise größere Möglichkeiten hinsichtlich der Fertigungsweise der Hochfrequenzschirmeinheit. Beispielsweise kann die Trägerschicht als Substrat für eine Aufbringung weiterer Schichten, insbesondere der ersten Leitschicht, der Isolationsschicht und der zweiten Leitschicht, dienen.

Unter einer Leitschicht ist hier in der Regel eine elektrisch leitfähige Schicht zu verstehen. Die erste und zweite Leitschicht umfasst also vorzugsweise jeweils zumindest ein elektrisch leitfähiges Material, d.h. ein Material mit einer hohen elektrischen Leitfähigkeit, die typischerweise bei einer Temperatur von 25 °C größer als 10³ S/m, insbesondere größer als 10⁶ S/m ist.

Die Trägerschicht und/oder die Isolationsschicht sind vorzugsweise elektrisch isolierende Schichten. Die Trägerschicht und/oder Isolationsschicht umfasst vorzuweise jeweils zumindest ein elektrisch isolierendes Material, d.h. ein Material mit einer niedrigen elektrischen Leitfähigkeit, die typischerweise bei einer Temperatur von 25 °C kleiner als 1 S/m, insbesondere kleiner als 10⁻³ S/m, insbesondere kleiner als 10⁻⁶ S/m ist.

Durch die räumliche Trennung der beiden Leitschichten durch die Isolationsschicht ergibt sich eine elektrische Kapazität, die zur Funktion der Hochfrequenzschirmeinheit beiträgt. Insbesondere kann ein hochfrequenter Strompfad, der durch etwaige Unterbrechungen in einer Leitschicht unterbrochen wird, über eine Kapazität in eine andere Leitschicht umgeleitet werden. Die Kapazität wird dabei insbesondere durch die Dicke und/oder die Dielektrizitätszahl, oft auch Permittivitätszahl oder relative Permittivität εᵣ genannt, der Isolationsschicht beeinflusst. Die Isolationsschicht kann hier also auch als Dielektrikum aufgefasst werden.

Herkömmliche Hochfrequenzschirmeinheiten sind üblicherweise aus drei Schichten, nämlich aus zwei Leitschichten und einer dazwischen liegenden Zwischenschicht aufgebaut, wobei die Zwischenschicht gleichzeitig als Isolationsschicht und als Trägerschicht dient. Die Zwischenschicht wirkt also einerseits elektrisch als Dielektrikum und andererseits mechanisch als mechanischer Träger. Durch diese Doppelfunktion der Zwischenschicht sind die Möglichkeiten, die Hochfrequenzschirmeinheit an die Anforderungen von Magnetresonanzvorrichtungen mit niedrigem Hauptmagnetfeld B₀ anzupassen, sehr eingeschränkt. Dieses Problem kann erfindungsgemäß durch eine Trennung der Doppelfunktion und der Zuordnung der beiden Funktionen zu jeweils einer eigenen Schicht, nämlich der Trägerschicht und der Isolationsschicht überwunden werden.

Vorzugsweise ist die erste Leitschicht eine Metallschicht, insbesondere eine Kupferschicht. Metalle, insbesondere Kupfer, weisen besonders hohe elektrische Leitfähigkeiten auf. Kupfer ist ferner gut verfügbar und lässt sich gut verarbeiten.

Vorzugsweise weist die erste Leitschicht eine Schichtdicke zwischen 1 und 50 µm, insbesondere 5 und 30 µm, insbesondere 9 und 18 µm auf. Mit solchen Schichtdicken kann vorteilhafterweise ein ausreichend geringer elektrischer Flächenwiderstand der Schicht erzielt werden. Somit kann insbesondere eine hohe Effizienz der Sendeantenne gewährleistet werden.

Vorzugsweise umfasst die Trägerschicht einen Verbundwerkstoff, der beispielsweise Epoxidharz und Glasfasergewebe umfasst. Insbesondere umfasst die Trägerschicht ein Trägermaterial für Leiterplatten, wie z.B. FR4. Mit Verbundwerkstoff können die gewünschten Eigenschaften der Trägerschicht besonders genau erzielt werden, insbesondere hinsichtlich der mechanischen Anforderungen. Verbundwerkstoffe weisen üblicherweise eine vergleichsweise hohe Stabilität bei geringem Gewicht auf. Insbesondere Leiterplattenmaterial ist zudem gut verfügbar und kostengünstig.

Vorzugsweise weist die Trägerschicht eine Schichtdicke von mindestens 0,1 mm, insbesondere mindestens 0,4 mm, insbesondere mindestens 0,7 mm auf. Mit einer Trägerschicht, die eine derartige Schichtdicke aufweist, kann die Hochfrequenzschirmeinheit besonders stabil und/oder wenig anfällig auf mechanische Beschädigungen ausgestaltet werden.

Vorzugsweise weisen die erste Leitschicht und/oder die zweite Leitschicht zumindest eine, insbesondere laterale, Unterbrechung auf, d.h. in der Schichtebene existieren ein oder mehrere Bereiche ohne elektrisch leitfähiges Material. Diese zumindest eine Unterbrechungen sind vorzugsweise länglich und/oder geschlitzt ausgebildet, z.B. als Trennschlitz. Die zumindest eine Unterbrechung kann insbesondere eine Schlitzstruktur bilden. Durch die zumindest eine Unterbrechung können Wirbelströme unterdrückt werden, die ansonsten in der Schicht fließen könnten. Damit kann eine Erwärmung der Hochfrequenzschirmeinheit minimiert werden.

Durch den vorgeschlagenen Schichtaufbau kann die Isolationsschicht, also die Schicht, durch die die erste Leitschicht und die zweite Leitschicht voneinander beabstandet wird, besonders dünn ausgeführt werden.

Interne Untersuchungen haben ergeben, dass bei Magnetresonanzvorrichtungen mit niedrigem Hauptmagnetfeld B₀ die Kapazität, die sich zwischen den beiden Leitschichten ausbildet, vorteilhafterweise reziprok zur Stärke des Hauptmagnetfeld B₀ angepasst werden sollte. Wird beispielsweise die Stärke des Hauptmagnetfeld B₀ um einen Faktor 3 von 1,5 T auf 0,5 T erniedrigt, wird vorteilhafterweise die Kapazität um den gleichen Faktor 3 erhöht. Eine Erhöhung der Kapazität kann insbesondere durch Verringerung der Schichtdicke erreicht werden, da üblicherweise eine indirekte Proportionalität zwischen der Schichtdicke und der Kapazität gegeben ist.

Da bei herkömmlichen Hochfrequenzschirmeinheiten die Zwischenschicht zwischen den beiden Leitschichten gleichzeitig auch die Stabilität der Hochfrequenzschirmeinheit gewährleisten muss, können üblicherweise keine Schichtdicken von weniger als 100 µm verwendet werden, da die Hochfrequenzschirmeinheit sonst zu anfällig für mechanische Beschädigungen wäre.

Vorzugsweise weist die Isolationsschicht eine Dielektrizitätszahl von mindestens 2, insbesondere mindestens 4, insbesondere mindestens 6 auf.

Gerade bei Magnetresonanzvorrichtungen mit niedrigem Hauptmagnetfeld B₀ ist eine Isolationsschicht mit einer hohen Dielektrizitätszahl vorteilhaft, um eine hohe Kapazität zwischen den beiden Leitschichten zu erzielen.

Vorzugsweise weist die Isolationsschicht Polymere auf. Polymerschichten können besonders dünn ausgestaltet werden, so dass damit hohe Kapazitäten zwischen den beiden Leitschichten erzielt werden können.

Vorzugsweise umfasst die Isolationsschicht Nanopartikel auf, die beispielsweise den Polymeren beigegeben sein können. Mit Nanopartikeln kann die Dielektrizitätszahl, und damit die Kapazität zwischen den beiden Leitschichten, vorteilhafterweise gezielt eingestellt werden. Vorzugsweise wird dadurch eine Dielektrizitätszahl der Isolationsschicht erreicht. Vorteilhafterweise werden dabei Nanopartikel eingesetzt, die eine Dielektrizitätszahl, vorzugsweise höher als 8, aufweisen. Insbesondere eigenen sich High-k-Dielektrika und/oder keramische Nanopartikel wie z.B. Aluminiumoxid.

Vorzugsweise ist die zweite Leitschicht zumindest teilweise durch ein Sprühverfahren auf der Isolationsschicht aufgebracht. Mittels Sprühverfahren kann die zweite Leitschicht besonders einfach und/oder dünn aufbracht werden.

Eine weitere Möglichkeit besteht darin, dass die zweite Leitschicht zumindest teilweise durch ein Druckverfahren, insbesondere ein Siebdruckverfahren, auf der Isolationsschicht aufgebracht ist. Mittels Druckverfahren kann die zweite Leitschicht besonders einfach und/oder dünn aufbracht werden.

Vorzugsweise ist durch das Druckverfahren eine Paste aufgebracht, die Kupfer und/oder Aluminium und/oder Kohlenstoffnanoröhrchen (engl. carbon nanotubes) enthält. Pasten lassen sich vorteilhafterweise besonders gut drucken. Kupfer und/oder Aluminium weisen vorteilhafterweise eine hohe elektrische Leitfähigkeit auf.

Vorzugsweise ist die zweite Leitschicht durch ein elektrochemisches Verfahren, insbesondere galvanisch, aufgedickt. Somit können vorteilhafterweise höhere Schichtdicken erreicht werden, um den elektrischen Flächenwiderstand der zweite Leitschicht zu minimieren.

Optional umfasst die Hochfrequenzschirmeinheit zumindest eine weitere Isolationsschicht und zumindest eine weitere Leitschicht. Dabei ist die zumindest eine weitere Isolationsschicht zwischen zwei Leitschichten angeordnet, d.h. der Schichtaufbau weist abwechselnd Leitschichten und Isolationsschichten auf. Zwischen zwei Leitschichten ist also vorzugsweise immer zumindest eine Isolationsschicht angeordnet.

Ein solcher Aufbau ermöglicht insbesondere größere Gestaltungsmöglichkeiten bei der Auslegung der Hochfrequenzschirmeinheit, so dass das Design der Hochfrequenzschirmeinheit noch besser optimiert werden kann. Insbesondere kann die Hochfrequenzschirmeinheit die Effizienz einer Sendeantenne somit weiter erhöhen und/oder mögliche Störungen können durch die Hochfrequenzschirmeinheit noch besser gedämpft werden.

Eine Ausführungsform der Hochfrequenzschirmeinheit sieht vor, dass die zumindest eine Isolationsschicht und die zumindest eine weitere Leitschicht eine andere laterale Ausdehnung aufweist als die Trägerschicht und/oder die erste Leitschicht und/oder die Isolationsschicht und/oder die zweite Leitschicht. Die zumindest eine Isolationsschicht und die zumindest eine weitere Leitschicht können lokal begrenzt ausgebildet sein. Somit können noch spezifischer lokale Gegebenheiten und/oder Anforderungen bei der Ausgestaltung der Hochfrequenzschirmeinheit berücksichtigt werden.

Ferner wird eine Magnetresonanzvorrichtung mit einer Hochfrequenzantenneneinheit und einer Gradientenspuleneinheit vorgeschlagen, wobei die Magnetresonanzvorrichtung eine vorab beschriebene Hochfrequenzschirmeinheit umfasst, der zwischen der Hochfrequenzantenneneinheit und der Gradientenspuleneinheit angeordnet ist. Durch die Hochfrequenzschirmeinheit kann insbesondere die Einkopplung von Störungen in die Hochfrequenzantenneneinheit vermindert werden.

Eine Ausführungsform der Magnetresonanzvorrichtung sieht vor, dass die Trägereinheit auf der der Gradientenspuleneinheit zugewandten Seite der Hochfrequenzschirmeinheit angeordnet ist. Oftmals wird bei der Fertigung die Hochfrequenzschirmeinheit zunächst in einem ersten Schritt auf die Gradientenspuleneinheit montiert und dann in einem zweiten Schritt die Hochfrequenzantenneneinheit auf die Hochfrequenzschirmeinheit und/oder Gradienteneinheit montiert. Damit weist die Ausführungsform, bei der die Trägereinheit auf der der Gradientenspuleneinheit zugewandten Seite der Hochfrequenzschirmeinheit angeordnet ist, insbesondere den Vorteil auf, dass dadurch ein leichterer Zugang zu den dann innen liegenden Leitschichten gegeben ist, um z.B. etwaige Kondensatoren und/oder Erdungsbänder zu verbinden.

Eine alternative Ausführungsform der Magnetresonanzvorrichtung sieht vor, dass die Trägereinheit auf der der Gradientenspuleneinheit abgewandten Seite der Hochfrequenzschirmeinheit angeordnet ist. Diese Ausführungsform weist insbesondere den Vorteil auf, dass durch die dann innen liegende Trägerschicht ein sehr guter mechanischer Schutz vor Beschädigungen gegeben ist.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
Fig. 1 ein Schema einer Magnetresonanzvorrichtung,
Fig. 2 ein Schema einer Hochfrequenzantenneneinheit und eine Gradientenspuleneinheit mit einer dazwischen liegenden Hochfrequenzschirmeinheit,
Fig. 3 ein Schema eine Hochfrequenzschirmeinheit in einer ersten Anordnungsvariante,
Fig. 4 ein Schema eine Hochfrequenzschirmeinheit in einer zweiten Anordnungsvariante,
Fig. 5 ein Schema einer ersten Leitschicht mit einem beispielhaften Trennschlitz,
Fig. 6 eine Schema eines Schichtaufbaus mit hochfrequenten Strompfaden,
Fig. 7 ein Schema einer Hochfrequenzschirmeinheit mit einer weiteren Isolationsschicht und einer weitere Leitschicht,
Fig. 8 ein Blockdiagramm eines Verfahren zur Herstellung einer Hochfrequenzschirmeinheit.

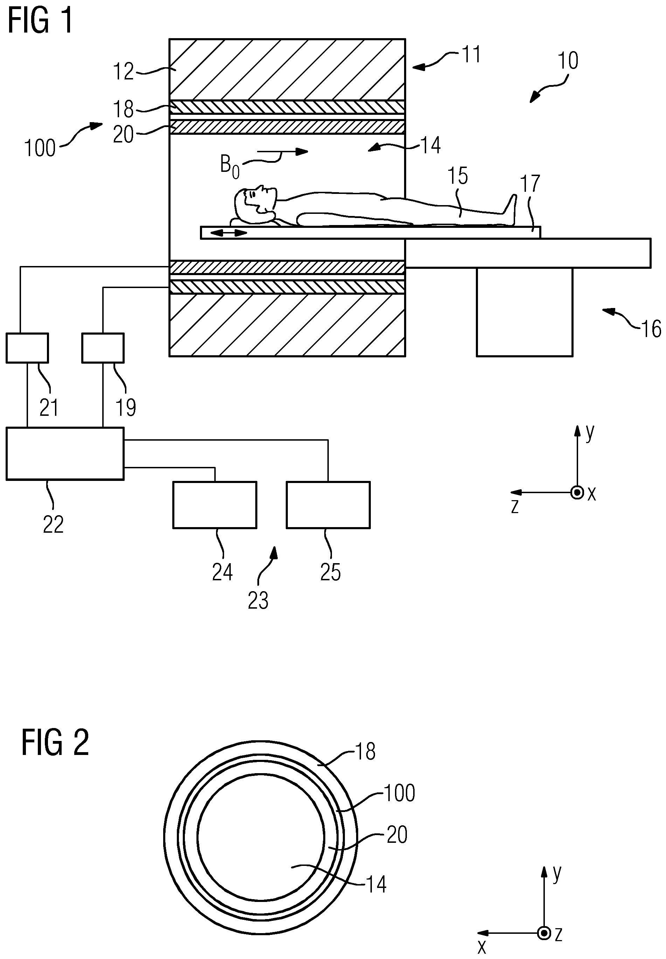

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds B₀ aufweist. Zudem umfasst die Magnetresonanzvorrichtung 10 einen Patientenaufnahmebereich 14 zu einer Aufnahme eines Patienten 15. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld B₀ einstellt, ausgelegt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum ein, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet.

Die Magnetresonanzvorrichtung 10 umfasst ferner eine Hochfrequenzschirmeinheit 100 zur Schirmung der Hochfrequenzantenneneinheit 20. Die Hochfrequenzschirmeinheit 100 ist zwischen der der Gradientenspuleneinheit 18 und der Hochfrequenzantenneneinheit 20 angeordnet. Wie auch in Fig. 2 gezeigt wird, sind die Hochfrequenzschirmeinheit 100, die Gradientenspuleneinheit 18 und die Hochfrequenzantenneneinheit 20 im vorliegenden Ausführungsbeispiel zylinderförmig ausgebildet. Dabei verlaufen die Hochfrequenzschirmeinheit 100, die Gradientenspuleneinheit 18 und die Hochfrequenzantenneneinheit 20 um eine z-Achse als Zylinderachse. Grundsätzlich ist jedoch eine davon abweichende Geometrie denkbar.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

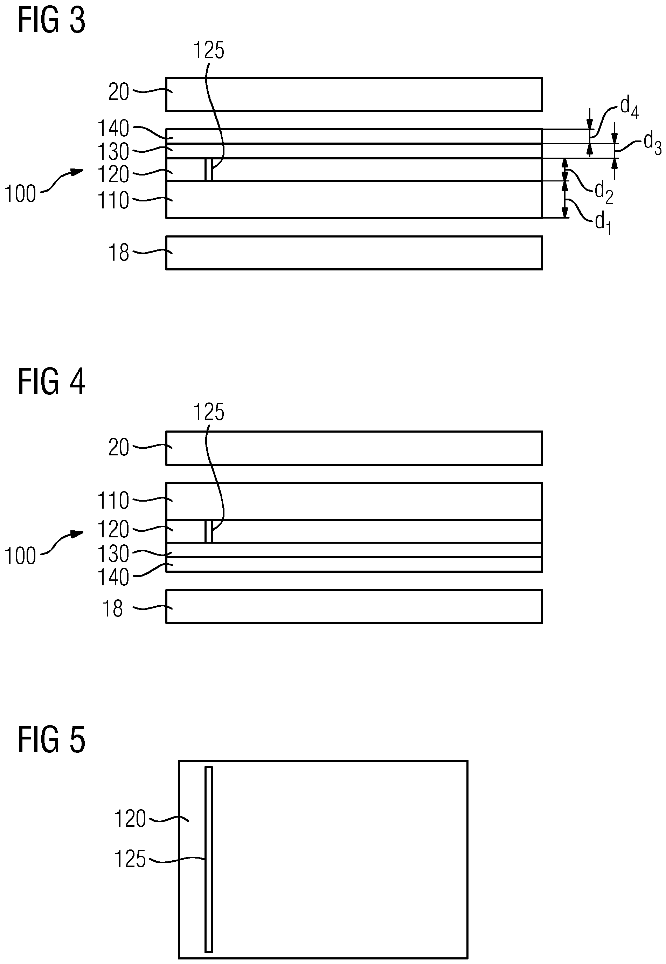

In den Fig. 3 und 4 ist jeweils ein Ausschnitt einer Hochfrequenzschirmeinheit 100 in unterschiedlichen Anordnungsvarianten relativ zu der Gradientenspuleneinheit 18 und der Hochfrequenzantenneneinheit 20 in Schnittansichten dargestellt. Vereinfachend wird die Hochfrequenzschirmeinheit 100 hier als ebene Abwicklung entlang des Umfangs um die z-Achse dargestellt. Die in Fig. 3 dargestellten Variante, bei der die Trägereinheit 110 auf der der Gradientenspuleneinheit 18 zugewandten Seite der Hochfrequenzschirmeinheit 100 angeordnet ist, ermöglicht einen leichteren Zugang zu der dann innen liegenden zweiten Leitschicht 140, um beispielsweise etwaige Bauteile zu verbinden. Bei der in Fig. 4 dargestellten Variante, bei der die Trägereinheit 110 auf der der Gradientenspuleneinheit 18 abgewandten Seite der Hochfrequenzschirmeinheit 100 angeordnet ist, ist hingegen ein sehr guter mechanischer Schutz vor Beschädigungen gegeben.

In Fig. 3 und 4 wird vereinfachend ein ebener Schichtaufbau gezeigt, der jedoch selbstverständlich insbesondere auch gekrümmt sein kann, wie beispielhaft in Fig. 1 oder 2 gezeigt. In beiden Fällen umfasst die Hochfrequenzschirmeinheit 100 eine Trägerschicht 110, eine erste Leitschicht 120, eine Isolationsschicht 130 und eine zweite Leitschicht 140. Die erste Leitschicht 120 ist zwischen der Trägerschicht 110 und der Isolationsschicht 130 angeordnet, und die Isolationsschicht 130 ist zwischen der ersten Leitschicht 120 und der zweiten Leitschicht 140 angeordnet.

Die Trägerschicht 110 und die erste Leitschicht 120 können beispielsweise ein Teil einer herkömmlichen Leiterplatte sein. Die Leiterplatte kann beispielsweise einen Verbundwerkstoff, insbesondere ein Leiterplattenträgermaterial wie FR4, als Trägerschicht 110 und eine Metallschicht, insbesondere eine Kupferschicht, als erste Leitschicht 110 aufweisen. Es ist insbesondere denkbar, dass die Leiterplatte nur einseitig, sondern auch beidseitig mit Metall, insbesondere Kupfer, beschichtet ist (hier nicht dargestellt), wobei dann eine der beiden Metallschichten als erste Leitschicht 120 fungiert.

Die Dicke der Trägerschicht d₁ kann dabei vorteilhafterweise überwiegend oder sogar ausschließlich unter mechanischen Gesichtspunkten ausgelegt werden, d.h. es müssen nicht wie bei konventionellen HF-Schirmen zusätzlich elektrische Aspekte berücksichtigt werden.

Die erste Leitschicht 120 und/oder zweite Leitschicht 140 kann optional eine Schlitzstruktur aufweisen. Dies ist hier beispielhaft durch den Trennschlitz 125 in der ersten Leitschicht 120 angedeutet, der eine Unterbrechung der ersten Leitschicht 120 darstellt. In Fig. 5 ist beispielhaft eine Draufsicht auf die erste Leitschicht 120 mit dem Trennschlitz 125 gezeigt. Natürlich kann die erste Leitschicht 120 und/oder zweite Leitschicht 140 noch weitere Unterbrechungen aufweisen. Die zumindest eine Unterbrechung ermöglicht eine Minimierung von Wirbelströmen in der ersten Leitschicht 120 und/oder zweiten Leitschicht 140.

Die Dicken d₂ und d₄ der Leitschichten 120, 140 werden vorteilhafterweise so gewählt, dass der elektrische Flächenwiderstand der Leitschichten 120, 140 ausreichend klein ist.

Die Isolationsschicht 130 kann z.B. aufgesprüht und/oder in einem Druckverfahren, insbesondere Siebdruckverfahren, auf die erste Leitschicht 120 aufgebracht werden. Dabei können besonders dünne Schichtdicken d₃ der Isolationsschicht 130 erreicht werden. Vorzugsweise liegt die Schichtdicke d₃ im Bereich von 20 µm, so dass sie damit deutlich geringer ist als die Zwischenschicht konventioneller HF-Schirme. Es sind dadurch Kapazitäten erreichbar, die im Vergleich zur konventionellen Fertigungsweisen, die in der Regel keine Schichtdicken unter 100 µm zulassen, um einen Faktor 5 höher sein können. Als Material für die Isolationsschicht 130 können beispielsweise Polymere zum Einsatz kommen. Die Dielektrizitätszahl der Isolationsschicht 130, die vorzugsweise zwischen ... liegt, kann beispielsweise durch Beimischung von Nanopartikeln gesteuert werden. Damit können Verlustfaktoren tanδ von etwa 0,02 erreicht werden, welche in der Größenordnung von FR4-Materialien liegt. Durch die geringere Schichtdicke d₃ sind die Verluste insgesamt aber weniger wirkungsvoll, da der ohmsche Verlustbeitrag linear mit der Schichtdicke d₃ abnimmt.

Vorzugsweise wird die zweite Leitschicht 140 nach einer Verfestigung der Isolationsschicht 130 aufgebracht. Dafür denkbar sind wieder Sprüh- und/oder Druckverfahren, insbesondere Siebdruckverfahren. Dabei ist besonders vorteilhaft, dass damit auch bereits eine gewünschte Schlitzstruktur erstellt werden kann. Zum Druck kann beispielsweise kupfer- oder aluminiumhaltige Paste verwendet werden. Um eine ausreichende elektrische Leitfähigkeit zu erzielen, kann die zweite Leitschicht weiter aufgedickt werden, insbesondere durch elektrochemische Verfahren wie etwa Galvanisierung.

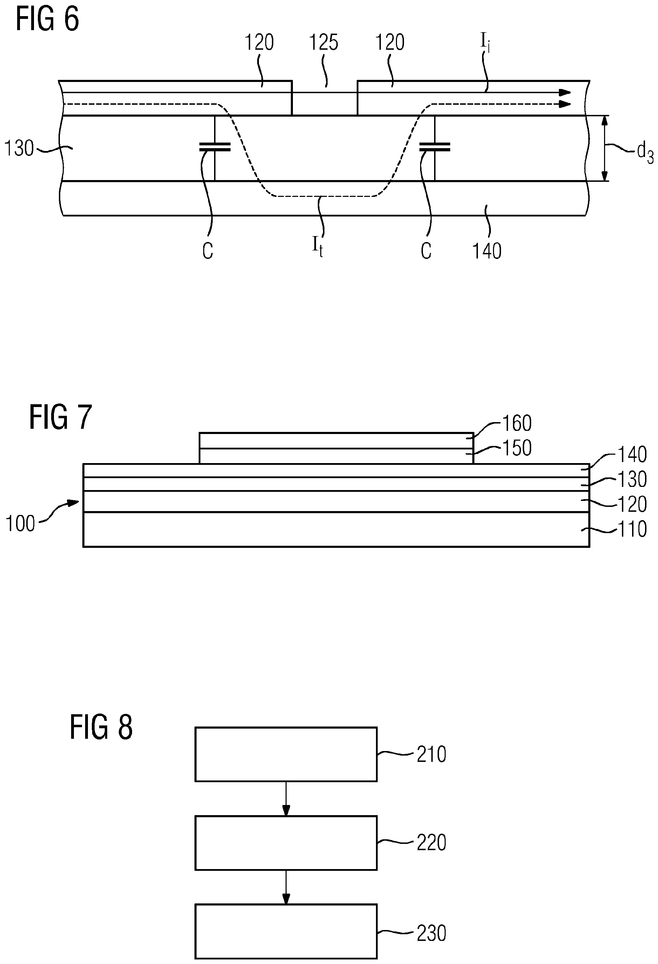

Anhand von Fig. 6 soll erläutert werden, auf welche Weise eine elektrische Kapazität zur Funktion der Hochfrequenzschirmeinheit 100 beiträgt. Dargestellt sind die erste Leitschicht 120 und die zweite Leitschicht 140, die durch die Isolationsschicht 130 getrennt sind. In diesem Beispiel umfasst die erste Leitschicht 120 eine Unterbrechung in Form eines Trennschlitzes 125, jedoch ist es analog ebenso möglich, dass die zweite Leitschicht 140 eine Unterbrechung aufweist.

In der ersten Leitschicht können im Allgemeinen Spiegelströme der Hochfrequenzantenneneinheit 20 fließen, die jedoch hier durch den Trennschlitz 125 am Fließen gehindert werden, d.h. ein idealer Strompfad Iᵢ wird durch den Trennschlitz 125 unterbrochen. Da es sich hierbei um einen hochfrequenten Strom handelt, kann dieser jedoch über die Kapazität C der Isolationsschicht 130 auf die zweite Leitschicht 140 bzw. von der zweiten Leitschicht 140 wieder in die erste Leitschicht 120 überkoppeln. Dadurch fließt der Strom teilweise kapazitiv über den tatsächlichen Strompfad Iₜ. Es wurde erkannt, dass in der Regel diese Koppelwirkung besser ist, je größer die Kapazität C ist. Üblicherweise gilt C∼εᵣ/d₃, so dass die Isolationsschicht 130 vorteilhafterweise eine hohe Dielektrizitätszahl εᵣ und/oder eine geringe Schichtdicke d₃ aufweist.

Fig. 7 zeigt eine Hochfrequenzschirmeinheit 100 mit einer weiteren Isolationsschicht 150 und einer weiteren Leitschicht 160. Ist ferner denkbar, dass die Hochfrequenzschirmeinheit 100 darüber hinaus noch zusätzliche weitere im Wechsel angeordnete Isolations- und Leitschichten umfasst.

Hier weisen die weitere Isolationsschicht 150 und die weitere Leitschicht 160 eine andere, nämlich geringere, laterale Ausdehnung auf als die Trägerschicht 110 und die erste Leitschicht 120 und die Isolationsschicht 130 und die zweite Leitschicht 140.

In Fig. 8 zeigt eine Übersicht über verschiedene Verfahrensschritte, die vorteilhafterweise bei der Herstellung der Hochfrequenzschirmeinheit durchgeführt werden können.

In Schritt 210 wird ausgehend von einer Trägerschicht 110 mit einer ersten Leitschicht 120 die Isolationsschicht 130 zumindest teilweise durch ein Sprühverfahren und/oder ein Druckverfahren auf die ersten Leitschicht 120 aufgebracht.

In Schritt 220 wird die zweite Leitschicht 140 zumindest teilweise durch ein Sprühverfahren und/oder ein Druckverfahren auf die Isolationsschicht 130 aufgebracht. Durch das Druckverfahren kann insbesondere eine Paste eine aufgebracht werden, die Kupfer und/oder Aluminium und/oder Kohlenstoffnanoröhrchen enthält.

In Schritt 230 wird die zweite Leitschicht 140 durch ein elektrochemisches Verfahren aufgedickt.

Zusammenfassend lässt sich festhalten, dass die vorgeschlagene Hochfrequenzschirmeinheit wie auch bisher zwei elektrisch leitfähige, möglicherweise auch geschlitzte, Lagen umfasst. Jedoch ergibt sich im Vergleich zu bisherigen Hochfrequenzschirmeinheiten insbesondere der Vorteil, dass eine deutlich dünnere Zwischenschicht zwischen den zwei elektrisch leitfähigen Lagen erzeugt werden können. Dadurch können die Eigenschaften der Hochfrequenzschirmeinheit insbesondere bei niedrigen B₀-Feldstärken deutlich verbessert werden.

Es wird abschließend noch einmal darauf hingewiesen, dass es sich bei den vorhergehend detailliert beschriebenen Verfahren sowie bei der dargestellten Erfassungsmustererzeugungseinheit und Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Hochfrequenzschirmeinheit (100) zur Schirmung einer Hochfrequenzantenneneinheit (20) einer Magnetresonanzvorrichtung (10), wobei die Hochfrequenzschirmeinheit (100) umfasst:
- eine Trägerschicht,
- eine erste Leitschicht (120),
- eine elektrisch isolierende Isolationsschicht (130) und
- eine zweite Leitschicht (140),
wobei die erste Leitschicht zwischen der Trägerschicht und der Isolationsschicht angeordnet ist,
wobei die Isolationsschicht (130) zwischen der ersten Leitschicht (120) und der zweiten Leitschicht (140) angeordnet ist,
**dadurch gekennzeichnet, dass**
sich durch die räumliche Trennung der beiden Leitschichten (120, 140) durch die Isolationsschicht (130) eine elektrische Kapazität ergibt;
wobei die Isolationsschicht (130) eine Schichtdicke (d₃) von höchstens 50 µm aufweist, wobei die Isolationsschicht (130) zumindest teilweise durch ein Sprühverfahren und/oder ein Druckverfahren auf der ersten Leitschicht aufgebracht ist.

2. Hochfrequenzschirmeinheit (100) nach einem der vorangehenden Ansprüche,
wobei die erste Leitschicht (120) und/oder zweite Leitschicht (140) zumindest eine Unterbrechung (125) aufweist.

3. Hochfrequenzschirmeinheit (100) nach einem der vorangehenden Ansprüche,
wobei die Isolationsschicht (130) eine Dielektrizitätszahl von mindestens 2 aufweist.

4. Hochfrequenzschirmeinheit (100) nach einem der vorangehenden Ansprüche,
wobei die Isolationsschicht (130) Polymere und/oder Nanopartikel umfasst.

5. Hochfrequenzschirmeinheit (100) nach einem der vorangehenden Ansprüche,
wobei die Hochfrequenzschirmeinheit (100) zumindest eine weitere Isolationsschicht (150) und zumindest eine weitere Leitschicht (160) umfasst.

6. Hochfrequenzschirmeinheit (100) nach Anspruch 5,
wobei die zumindest eine weitere Isolationsschicht (150) und die zumindest eine weitere Leitschicht (160) eine andere laterale Ausdehnung aufweist als die Trägerschicht (110) und/oder die erste Leitschicht (120) und/oder die Isolationsschicht (130) und/oder die zweite Leitschicht (140).

7. Hochfrequenzschirmeinheit (100) nach einem der vorangehenden Ansprüche,
wobei die erste Leitschicht (120) eine Kupferschicht ist.

8. Magnetresonanzvorrichtung (10) mit einer Hochfrequenzantenneneinheit (20) und einer Gradientenspuleneinheit (18), wobei die Magnetresonanzvorrichtung (10) eine Hochfrequenzschirmeinheit (100) nach einem der Ansprüche 1 bis 6 umfasst, die zwischen der Hochfrequenzantenneneinheit (20) und der Gradientenspuleneinheit (18) angeordnet ist.

## Claims

1. Radio-frequency shielding unit (100) for shielding a radio-frequency antenna unit (20) of a magnetic resonance apparatus (10), wherein the radio-frequency shielding unit (100) comprises:
- a support layer;
- a first conducting layer (120);
- an electrically isolating insulating layer (130); and
- a second conducting layer (140);
wherein the first conducting layer is arranged between the support layer and the insulating layer,
wherein the insulating layer (130) is arranged between the first conducting layer (120) and the second conducting layer (140),
**characterised in that**
an electrical capacitance is produced by the spatial separation of the two conducting layers (120, 140) by the insulating layer (130);
wherein the insulating layer (130) has a layer thickness (d₃) of 50 µm maximum,
wherein the insulating layer (130) is applied to the first conducting layer at least in part by a spray-coating process and/or a printing process.

2. Radio-frequency shielding unit (100) according to one of the preceding claims,
wherein the first conducting layer (120) and/or second conducting layer (140) comprise at least one discontinuity (125) .

3. Radio-frequency shielding unit (100) according to one of the preceding claims,
wherein the insulating layer (130) has a dielectric constant of at least 2.

4. Radio-frequency shielding unit (100) according to one of the preceding claims,
wherein the insulating layer (130) comprises polymers and/or nanoparticles.

5. Radio-frequency shielding unit (100) according to one of the preceding claims,
wherein the radio-frequency shielding unit (100) comprises at least one additional insulating layer (150) and at least one additional conducting layer (160).

6. Radio-frequency shielding unit (100) according to claim 5, wherein the at least one additional insulating layer (150) and the at least one additional conducting layer (160) have a different lateral extent compared with the support layer (110) and/or the first conducting layer (120) and/or the insulating layer (130) and/or the second conducting layer (140).

7. Radio-frequency shielding unit (100) according to one of the preceding claims,
wherein the first conducting layer (120) is a copper layer.

8. Magnetic resonance apparatus (10) having a radio-frequency antenna unit (20) and a gradient coil unit (18), wherein the magnetic resonance apparatus (10) comprises a radio-frequency shielding unit (100) according to one of claims 1 to 6, which is arranged between the radio-frequency antenna unit (20) and the gradient coil unit (18).

## Revendications

1. Unité (100) de protection de haute fréquence pour la protection d'une unité (20) d'antenne de haute fréquence d'un système (10) de résonnance magnétique, l'unité (10) de protection de haute fréquence comprenant :
- une couche de support,
- une première couche (120) conductrice,
- une couche (130) isolante isolante du point de vue électrique et
- une deuxième couche (140) conductrice,
dans laquelle la première couche conductrice est disposée entre la couche de support et la couche isolante,
dans laquelle la couche (130) isolante est disposée entre la première couche (120) conductrice et la deuxième couche (140) conductrice,
**caractérisée en ce que**
une capacité électrique est produite par la séparation dans l'espace des deux couches (120, 140) conductrices par la couche (130) isolante ;
dans laquelle la couche (130) isolante a une épaisseur (d₃) d'au plus 50 µm,
dans laquelle la couche (130) isolante est déposée sur la première couche conductrice, au moins en partie, par un procédé de pulvérisation et/ou par un procédé d'impression.

2. Unité (100) de protection de haute fréquence suivant l'une des revendications précédentes,
dans laquelle la première couche (120) conductrice et/ou la deuxième couche (140) conductrice a au moins une interruption (125).

3. Unité (100) de protection de haute fréquence suivant l'une des revendications précédentes,
dans laquelle la couche (130) isolante a une permittivité relative d'au moins 2.

4. Unité (100) de protection de haute fréquence suivant l'une des revendications précédentes,
dans laquelle la couche (130) isolante comprend des polymères et/ou des nanoparticules.

5. Unité (100) de protection de haute fréquence suivant l'une des revendications précédentes,
dans laquelle l'unité (100) de protection de haute fréquence comprend au moins une autre couche (150) isolante et au moins une autre couche (160) conductrice.

6. Unité (100) de protection de haute fréquence suivant la revendication 5,
dans laquelle la au moins une autre couche (150) isolante et la au moins une autre couche (160) conductrice ont une étendue latérale autre que la couche (110) de support et/ou que la première couche (120) conductrice et/ou que la couche (130) isolante et/ou que la deuxième couche (140) conductrice.

7. Unité (100) de protection de haute fréquence suivant l'une des revendications précédentes,
dans laquelle la première couche (120) conductrice est une couche de cuivre.

8. Système (10) de résonnance magnétique, comprenant une unité (20) d'antenne de haute fréquence et une unité (18) de bobine de gradient, le système (10) de résonnance magnétique comprenant une unité (100) de protection de haute fréquence suivant l'une des revendications 1 à 6, qui est disposée entre l'unité (20) d'antenne de haute fréquence et l'unité (18) de bobine de gradient.
